# EUROPÄISCHE PATENTSCHRIFT

(11) **EP 2 849 645 B1**
(45) Veröffentlichungstag und Bekanntmachung des Hinweises auf die Patenterteilung: **16.11.2022**
(21) Anmeldenummer: 13722289.9
(22) Anmeldetag: 14.05.2013
(51) Int. Cl.: A61B 5/11

(54) **VORRICHTUNG UND VERFAHREN ZUR BESTIMMUNG VON FEHLSTELLUNGEN IM AUFBAU VON PROTHESEN**
DEVICE AND METHOD FOR DETERMINING AN INCORRECT POSITIONING IN THE ALIGNMENT OF PROSTHESES
DISPOSITIF ET PROCÉDÉ POUR DÉTERMINER LES ERREURS DE POSITIONNEMENT DANS LA RÉALISATION DE PROTHÈSES

(30) Priorität: 14.05.2012 DE 102012009507
(43) Veröffentlichungstag der Anmeldung: 25.03.2015
(73) Patentinhaber: Ottobock SE & Co. KGaA, 37115 Duderstadt (DE)
(72) Erfinder: PUSCH, Martin, 37115 Duderstadt (DE)
(74) Vertreter: Friedrich, Andreas
(86) Internationale Anmeldenummer: PCT/EP2013/001404
(87) Internationale Veröffentlichungsnummer: WO 2013/170945

(56) Entgegenhaltungen:
- WO-A1-2009/114909
- WO-A2-2012/050908
- US-A1- 2010 131 113
- US-A1- 2011 313 545
- ANDRES R O ET AL: "Prosthetic alignment effects on gait symmetry: a case study", CLINICAL BIOMECHANICS, BUTTERWORTH SCIENTIFIC LTD, GUILDFORD, GB, Bd. 5, Nr. 2, 1. Mai 1990 (1990-05-01), Seiten 88-96, XP026180847, ISSN: 0268-0033, DOI: 10.1016/0268-0033(90)90043-6 [gefunden am 1990-05-01]

## Beschreibung

Die Erfindung betrifft ein Verfahren zur Bestimmung von Fehlstellungen eines Prothesenfußes relativ zu einem Unterschenkelrohr im Aufbau von Prothesen der unteren Extremitäten mit folgenden Schritten: Ermittelung von Inertialmessdaten und/oder der daraus abgeleiteten Größen einer versorgten Extremität über zumindest einen Inertialsensor, der an einer Komponente der Prothese der unteren Extremität angeordnet und mit einem Komparator gekoppelt ist, über zumindest einen Gangzyklus und Vergleichen der ermittelten Inertialmessdaten und/oder der daraus ermittelten Größen mit Sollwerten in dem Komparator.

Ein solches Verfahren ist beispielsweise aus der US 2010/131113 A1 und der US 2011/313545 A1 bekannt.

Prothesen dienen im Allgemeinen dazu, die Form und insbesondere die Funktion nicht vorhandener oder nicht mehr vorhandener Gliedmaßen zu ersetzen. Während die aus rein kosmetischen Beweggründen angefertigten Prothesen keine funktionalen Aufgaben zu erfüllen haben, sehen Prothesen an den unteren und oberen Extremitäten regelmäßig die Übernahme komplexer Aufgaben vor. Handprothesen bilden die Funktion einer natürlichen Hand in weitem Umfang nach, wobei insbesondere die Steuerung der motorischen Antriebe hohen Aufwand erfordert.

Prothesen der unteren Extremitäten bestehen regelmäßig aus Prothesenfüßen, einem Unterschenkelrohr und gegebenenfalls einem Prothesenkniegelenk und werden häufig über Schäfte an dem verbliebenen Stumpf befestigt. Die Konstruktion von Prothesen der unteren Extremitäten orientiert sich an den Bedürfnissen und Fähigkeiten der Patienten und reicht von einem einachsigen Sperrkniegelenk bis zu rechnergesteuerten Prothesen, die auf der Basis von Sensorwerten die Dämpfungseigenschaften verändern und Antriebe zu- oder abschalten.

Ein wesentlicher Gesichtspunkt bei der prothetischen Versorgung ist die richtige Anpassung der Prothese an den Prothesennutzer und die korrekte Zuordnung der einzelnen Prothesenkomponenten zueinander. Diese Ausrichtung der Prothesenkomponenten zueinander und an dem Patienten wird in der Orthopädietechnik der Prothesenaufbau oder der Aufbau der Prothese genannt. Dabei erfolgt die Anpassung und gegebenenfalls Optimierung der Einstellungen von Ober- oder Unterschenkelprothesen im Rahmen des Versorgungsprozesses durch einen Orthopädietechniker individuell für einen Patienten. Der Orthopädietechniker orientiert sich dabei an Aufbauanleitungen sowie an seiner Beobachtung oder den Eindrücken des Patienten.

Zur Beurteilung des Aufbaus kann ein Orthopädietechniker zur statischen Analyse auf ein Anzeigesystem zurückgreifen, das in der DE 44 01 036 C2 beschrieben ist. Das Anzeigesystem sieht eine Messplatte sowie eine Projektionseinrichtung zur Visualisierung einer lotrechten Messlinie auf dem Körper einer auf der Messplatte stehenden Person vor, wobei die Messplatte mit über eine Schaltung miteinander verbundenen Drucksensoren bestückt ist, über die ein Antrieb gesteuert wird, der in einer Ebene parallel zu der Messplatte ein optisches System dergestalt verschiebt, dass ein Lichtstrahl den momentanen Schwerpunkt der Person anzeigt.

Aus der US 2008/285805 A1 ist ein System zur Nachverfolgung von Bewegungen des menschlichen Körpers bekannt, bei dem über 3D-lnertialwinkelsensoren die Bewegungen der Extremitäten aufgenommen und drahtlos an eine Auswerteeinheit übermittelt werden.

Die WO 2010/120402 A1 betrifft ein Steuerungssystem zur Steuerung einer zugeordneten Vorrichtung mit einer inertialen Messeinheit zur Ermittlung der Orientierung dreier Achsen mit einer Recheneinheit zur Auswertung der Sensordaten. Über eine Kommunikationseinheit steht das Sensormodul mit der zugeordneten Einrichtung in Verbindung und kann darüber gesteuert werden. Rückschlüsse auf einen Prothesenaufbau ergeben sich daraus nicht.

Aufgabe der vorliegenden Erfindung ist es, ein Verfahren und eine Vorrichtung bereitzustellen, mit denen es möglich ist, auf einfache Art und Weise durch ganganalytische Messdaten den Prothesenaufbau beurteilen zu können, so dass nach entsprechender Korrektur ein verbesserter Prothesenaufbau für den Patienten bereitgestellt werden kann.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen aufgeführt.

Das Verfahren zur Bestimmung von Fehlstellungen im Aufbau von Prothesen der unteren Extremitäten sieht vor, dass eine Ermittlung von Inertialmessdaten und/oder daraus abgeleiteten Größen einer versorgten Extremität mit zumindest einem Inertialsensor über zumindest einen Gangzyklus durchgeführt wird. Eine versorgte Extremität ist eine Extremität, an der eine Protheseneinrichtung befestigt ist oder die durch eine Protheseneinrichtung ersetzt wird. Bei transtibialen Prothesen sind die typischen Komponenten ein Prothesenfuß und ein Unterschenkelschaft, gegebenenfalls ein Pylon, an dem der Prothesenfuß befestigt ist, bei transfemoralen Prothesen ist ein Oberschenkelschaft zur Aufnahme eines Oberschenkelstumpfes, ein Prothesenkniegelenk, ein Unterschenkrohr und ein Prothesenfuß vorgesehen. Nachdem beispielsweise der Absolutwinkel, die Beschleunigungen, Drehraten oder Gierraten einer Extremität, beispielsweise am Unterschenkelrohr, Unterschenkelschaft und am Oberschenkelschaft gemessen wurden, und ggf. daraus abgeleitete Größen wie Geschwindigkeit, Bewegungsrichtung und die Orientierung der Komponenten im Raum und zueinander sowie deren Bewegung im Raum und zueinander bestimmt oder errechnet wurden, werden die ermittelten Inertialmessdaten und/oder daraus abgeleiteten Größen mit Sollwerten und/oder mit ermittelten Messdaten oder abgeleiteter Größen für die korrespondierende unversorgte Extremität verglichen. Die Sollwerte können in einem Speicher abgelegt sein und auf Erfahrungswerten oder Werten, die an dem zu versorgenden Patienten aufgenommen worden sind, basieren. Die ermittelten Inertialmessdaten, z.B. Winkelwerte, Beschleunigungen, Orientierungen oder Richtungen, können sich dabei auf Größen in unterschiedlichen Ebenen beziehen. Beispielsweise können Winkel in der Sagittalebene ermittelt und verglichen werden, um festzustellen, wie groß die Schrittlänge ist, wie weit der Unterschenkel und damit das Knie beim Auftreten eingebeugt wird oder wie weit der Unterschenkel in der Schwungphase nach hinten durchschwingt. Alternativ zu dem Vergleich der ermittelten Messdaten und/oder abgeleiteter Größen an der versorgten Extremität mit gespeicherten Sollwerten ist es möglich, an den korrespondierenden Stellen der unversorgten, also gesunden Extremität korrespondierende Messdaten und/oder abgeleitete Größen zu ermitteln, z.B. Absolutwinkel über Inertialwinkelsensoren oder Beschleunigungen über Beschleunigungssensoren zu messen und dem Vergleich zugrunde zu legen. Wird beispielsweise über einen Gangzyklus eine abweichende Orientierung des Oberschenkels, des Unterschenkels oder des Kniewinkels relativ zu der kontralateralen Seite ermittelt, kann davon ausgegangen werden, dass kein harmonisches Gangbild vorliegt, wobei auf der Grundlage der Abweichungen dann auf die vorzunehmenden Veränderungen geschlossen werden kann. Je nach Abweichung wird es notwendig sein, die einzelnen Prothesenkomponenten zueinander und an dem Prothesennutzer neu auszurichten, wozu die Ermittlung der Inertialmessdaten und/oder der daraus abgeleiteten Größen und der Vergleich mit Sollwerten und/oder Messwerten oder daraus abgeleiteter Größen korrespondierender Extremitäten wertvolle Hinweise geben.

Inertialsensoren messen neben dem Absolutwinkel auch die linearen Beschleunigungen der jeweiligen Komponente, die entlang der Achsen eines Bezugssystems auftreten, ebenso wie die Drehraten oder Winkelgeschwindigkeiten um die jeweiligen Achsen, um daraus die Rotationsbewegungen ableiten zu können.

Eine Ausgestaltung der Erfindung sieht vor, dass als Inertialsensoren insbesondere Absolutwinkelsensoren eingesetzt werden, über die die Absolutwinkel der jeweiligen Komponente oder Extremität ermittelt wird. Die Ermittelung kann unmittelbar durch die Sensoren erfolgen, so dass eine Umrechnung nicht vorgenommen werden muss, wie es zur Ermittelung der abgeleiteten Größe erforderlich ist.

Sowohl an der mit der Prothese versorgten als auch an der unversorgten korrespondierenden Extremität kann der jeweilige Inertialmesswert, z.B. die Beschleunigung oder der Absolutwinkel ermittelt werden, wobei auch bei transtibialer Versorgung Messwerte und daraus abgeleitete Größen an dem Oberschenkel, z.B. der Oberschenkelwinkel, die Bewegungsrichtung, Beschleunigung oder Beschleunigungsrichtung, ermittelt werden kann. Die Anbringung an der unversorgten Extremität ist für einen unmittelbaren Vergleich zwischen den Extremitäten notwendig.

Erfindungsgemäß ist vorgesehen, dass der Kniewinkel aus den gemessenen Absolutwinkeln des Unterschenkels und des Oberschenkels bestimmt und mit Sollwerten und/oder dem ermittelten Kniewinkel des unversorgten Beines über den jeweiligen Gangzyklus oder einen Mittelwert mehrerer Gangzyklen verglichen wird. Der Beginn eines Gangzyklus' kann anhand des charakteristischen Winkelverlaufes beim Gehen in der Ebene, beispielsweise anhand des Kniewinkelverlaufs, ermittelt werden. Dadurch ist es möglich, auf zusätzliche Sensoren, die beispielsweise Kräfte oder Momente messen, zu verzichten. Es besteht also die Möglichkeit, aufgrund einer reinen Winkelmessung von Extremitäten nach dem Anlegen der Prothese aufgrund gangdynamischer Messungen festzustellen, ob ein Prothesenaufbau dem angestrebten Ergebnis entspricht, insbesondere ob ein harmonisches Gangbild für einen Patienten möglich ist, ohne dass dabei auf eine reine Beobachtung des Bewegungsablaufes durch einen Orthopädietechniker zurückgegriffen werden muss. Vielmehr ist es möglich, auf der Grundlage objektiver Messwerte Rückschlüsse zu ziehen, ob der Prothesenaufbau korrekt ist, so dass es möglich ist, Winkelverläufe über einen Gangzyklus darzustellen und mit einer physiologischen Sollkurve oder dem korrespondierenden Winkelverlauf des unversorgten Beines zu vergleichen.

Eine Weiterbildung der Erfindung sieht vor, dass eine Abweichungsmeldung bei Abweichungen der ermittelten Inertialmessdaten oder der daraus abgeleiteten Größen von den abgelegten Sollwerten und/oder Messdaten oder daraus abgeleiteter Größen der korrespondierenden unversorgten Extremität ausgegeben wird. Alternativ zu einer Abweichungsmeldung kann auch eine Bestätigungsmeldung bei Werten innerhalb der Sollwerte bzw. innerhalb eines zulässigen Streubereiches um einen Sollwert oder um ermittelte Messdaten oder daraus abgeleiteter Größen für die korrespondierende unversorgte Extremität ausgegeben werden, so dass der Orthopädietechniker ebenso wie der Patient eine Rückmeldung erhält, ob der statische Prothesenaufbau korrekt ist oder nicht.

Neben den Inertialmessdaten, z.B. den Absolutwinkeln der unteren Extremität, also des Fußes, des Unterschenkels und des Oberschenkels, ist es vorgesehen, dass ein Beckenwinkel innerhalb der Frontalebene über zumindest einen Inertialwinkelsensor ermittelt und bei Überschreiten eines Grenzwertes eine Abweichungsmeldung ausgegeben wird. Die Verkippung des Beckenwinkels innerhalb der Frontalebene beim Gehen ist ein Indiz für das Trendelenburg-Hinken. Überschreitet die Beckenkippung einen definierten Winkel während des Gehens innerhalb der Frontalebene, dient dies für den Orthopädietechniker als Hinweis darauf, dass eine weitere Adduktion des Schaftes erfolgen muss.

Sinnvollerweise werden die Inertialmessdaten, insbesondere Absolutwinkel, über mehrere Gangzyklen ermittelt, beispielsweise auf einem Laufband oder in einer Ganganalyse-Einrichtung, um eine möglichst breite Datenbasis für die Beurteilung des statischen Aufbaus der Prothese, für die Optimierung des Prothesenaufbaues, der Einstellung der Prothese, der Komponentenauswahl sowie der Dokumentation zu erlangen. Neben der Ermittelung der Inertialmessdaten über mehrere Gangzyklen können auch die daraus abgeleiteten Größen über mehrere Gangzyklen bestimmt und genutzt werden. Auch ist es möglich, anhand der Messwerte oder der daraus abgeleiteten Größen den Patienten besser zu informieren und instruieren, so dass aus einem verbesserten Verständnis für die Zusammenhänge von Aufbau und Verhalten der Prothese während der Benutzung eine verbesserte Akzeptanz durch den Patienten und eine verbesserte Ausnutzung der Prothesen erreicht werden kann.

Über die Inertialwinkelbestimmung ist es auch möglich festzustellen, ob der Außenrotationswinkel der Knieachse korrekt eingestellt ist. Wird während der Schwungphase eine Rotationsschwingung des versorgten Unterschenkels festgestellt, ist dies ein Hinweis darauf, dass die Knieachse nicht orthogonal zu der Bewegungsrichtung des Oberschenkels ausgerichtet ist, so dass der Unterschenkel eine dynamische Unwucht erfährt. Diese dynamische Unwucht kann über die Schritterkennung und die zeitliche Zuordnung der Messwerte innerhalb der Gangphase erfolgen, wird nämlich während der Schwungphase eine hohe Schwingfrequenz des gebeugten Unterschenkels gemessen, ist dies ein Hinweis für eine unzutreffende Ausrichtung der Gelenkachse.

Eine Vorrichtung zur Durchführung eines hier beschriebenen Verfahrens mit zumindest einem Inertialsensor sieht vor, dass dieser an einer Komponente einer Prothese der unteren Extremität angeordnet und mit einem Komparator gekoppelt ist, in dem die ermittelten Inertialmessdaten, z.B. Winkel, Beschleunigung, Richtung mit Sollwerten und/oder Vergleichswerten verglichen werden, wobei die Sollwerte in dem Komparator abgelegt sind oder eine entsprechende Datenbank mit dem Komparator verbunden ist. Die Vergleichswerte beziehen sich auf Werte des unversorgten Beines des Patienten, die ebenfalls in dem Komparator abgelegt oder in einer Datenbank für diesen zugänglich gemacht sind. Die Zugänglichmachung kann beispielsweise über eine Funkverbindung stattfinden.

Zur Ermittlung der Vergleichswerte an der unversorgten Extremität ist es vorgesehen, dass zumindest ein Inertialsensor an der unversorgten Extremität befestigt ist, um Vergleichswerte zu ermitteln. Sind mehrere Inertialsensoren an der versorgten Extremität vorgesehen, beispielsweise am Unterschenkel und am Oberschenkel, ist es vorteilhaft, wenn korrespondierende Sensoren an der unversorgten Extremität an korrespondierenden Stellen angeordnet sind. Auch diese Sensoren sind dann mit dem Komparator verbunden, beispielsweise verkabelt oder über eine Funkstrecke.

Die Ausgabeeinrichtung für die ermittelten und gegebenenfalls verglichenen Werte oder eine Abweichungsmeldung, wenn die Werte für die versorgte Seite nicht den Sollwerten oder Vergleichswerten entsprechen, ist ebenfalls mit dem Komparator verbunden und kann als akustische oder optische Ausgabeeinrichtung ausgebildet sein. Dadurch ist es möglich, dass während des Gehens und der Messung der Messdaten sowohl für den Nutzer als auch für den Orthopädietechniker eine unmittelbare Rückmeldung vorliegt, ob ein harmonisches Gangbild vorliegt, ob die dynamischen Winkelverläufe den Vorgaben oder Sollwerten entsprechen, ob andere Messdaten und/oder abgeleitete Größen den Vorgaben oder Vergleichswerten und ob der Prothesenaufbau zutreffend eingestellt ist oder nicht.

Nachfolgend wird die Erfindung anhand der Figuren näher erläutert. Es zeigen:
- Figur 1 -: eine schematische Darstellung einer Prothese mit einem weit anterior eingestellten Fuß;
- Figur 2 -: einen Fersenauftritt mit einem Fuß weit posterior;
- Figur 3 -: eine Zehenablösung mit einem Fuß weit anterior; sowie
- Figur 4 -: eine Zehenablösung mit einem Fuß weit posterior.

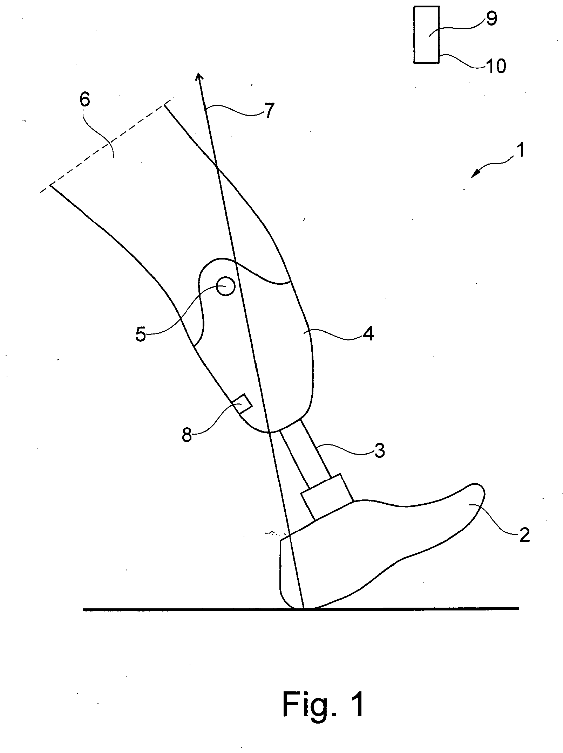

In der Figur 1 ist eine Prothese 1 mit einem Prothesenfuß 2, einem proximal daran befestigten Unterschenkelrohr 3 und einem Schaft 4 gezeigt, der eine Verbindung zu der verbliebenen unteren Extremität 6 herstellt. Der Schaft 4 überragt das noch verbliebene Kniegelenk, so dass eine Kniedrehachse 5 teilweise überdeckt wird. Im dargestellten Ausführungsbeispiel ist eine Transtibialprothese dargestellt, die kein Prothesenkniegelenk aufweist. Grundsätzlich gelten die nachfolgenden Ausführungen auch für transfemurale Prothesen mit einem Prothesenkniegelenk.

Der Prothesenfuß 2 ist relativ verschieblich zu dem Unterschenkelschaft 3 angeordnet, beispielsweise über einen nicht dargestellten Verschiebeadapter oder eine Langlochführung, so dass neben einer Rotation um die Längserstreckung des Unterschenkelrohres 3 auch eine Verschiebung des Prothesenfußes 2 anterior in Gehrichtung oder posterior entgegen der Gehrichtung möglich ist. An dem Schaft 4 ist ein Inertialsensor 8 in Gestalt eines Inertialwinkelsensors angeordnet oder eingearbeitet, über den der Absolutwinkel der Prothese, im vorliegenden Fall des Schaftes 4 und des Unterschenkelrohres 3, relativ zur Vertikalen ermittelt werden kann. Als Inertialsensoren sind insbesondere Winkelsensoren vorgesehen, die den Absolutwinkel relativ zu der Gravitationsrichtung ermitteln, ebenfalls können Beschleunigungssensoren und/oder Drehratensensoren vorgesehen sein, um die Inertialmessdaten aufzunehmen.

Durch die Anbringung weiterer Inertialsensoren, beispielsweise am Oberschenkel und an der Hüfte, ist es möglich, bestimmte Körpersegmentwinkel und/oder - beschleunigungen dynamisch zu messen. Bei den Winkels sind dies insbesondere die Orientierung des Oberschenkels, die Orientierung des Unterschenkels, die Orientierung der Hüfte sowie die Beugung des Knies aus dem Differenzwinkel zwischen dem Oberschenkelwinkel und dem Unterschenkelwinkel in der Sagittalebene. Anhand dieser gemessenen oder errechneten Winkel lässt sich eine Schritterkennung verwirklichen, die eine zeitliche Zuordnung der gemessenen Winkelwerte zu der jeweiligen Gangphase zulässt. Somit ist es möglich, eine zeitlich aufgelöste Winkelerfassung für die Standphase und die Schwungphase während des Gehens zu erreichen. Dadurch ist es möglich, die Winkelverläufe der jeweiligen Körpersegmente über einen Gangzyklus oder auch über mehrere Gangzyklen darzustellen und diese Winkelverläufe mit denen einer vorgegebenen Sollkurve oder mit Werten zu vergleichen, die an dem kontralateralen, unversorgten Bein gemessen wurden. Auch ist es möglich, die linearen Beschleunigungen zu messen, die entlang der Achsen eines Bezugssystems auftreten, ebenso wie die Drehraten oder Winkelgeschwindigkeiten um die jeweiligen Achsen, um daraus die Rotationsbewegungen ableiten zu können.

Ein kritischer Parameter bei der Anpassung von transtibialen Prothesen ist eine ausreichende Kniebeugung und eine ausreichende Kniestreckung nach dem Fersenauftritt. Dieser Parameter kann maßgeblich durch die Positionierung des Prothesenfußes 2 relativ zu dem Unterschenkelrohr 3 und damit zu dem Schaft 4 beeinflusst werden. Eine Änderung der Positionierung des Prothesenfußes 2 in anterior-posterior-Richtung führt zu einer Veränderung des Verlaufes der Bodenreaktionskraft 7 beim Auftreten, Abrollen und am Ende der Standphase bei der Zehenablösung. Diese Änderung des Verlaufes hat eine Änderung des wirksamen Kniemomentes um die Knieachse 5 zur Folge, welches je nach Lage der Bodenreaktionskräfte 7 relativ zu der Gelenkachse 5 streckend oder beugend wirkt.

In der Figur 1 ist der Prothesenfuß 2 maximal anterior verlagert, so dass bei einem Fersenauftritt zu Beginn der Standphase der Vektor der Bodenreaktionskraft 7 anterior der Kniedrehachse 5 orientiert ist. Dies führt zu einem streckenden Moment um die Kniedrehachse, so dass beim Fersenauftritt keine oder nur eine geringe Kniebeugung ermittelt werden kann. Wenn der Patient beim Geradeausgehen in der Ebene nach dem Fersenauftritt keine Kniebeugung zeigt, sondern eine Streckung nach dem Fersenauftritt und damit einhergehend aufgrund der nicht vorhandenen Einfederung eine Beckenverkippung in der Frontalebene detektiert wird, kann ein Orthopädietechniker darauf schließen, dass der Prothesenfuß 2 zu weit anterior angeordnet ist, so dass eine Verstellung in posterior-Richtung erforderlich ist. Diese Einstellung kann schrittweise erfolgen, bis der gewünschte Kniewinkel beim normalen Gehen erreicht wird.

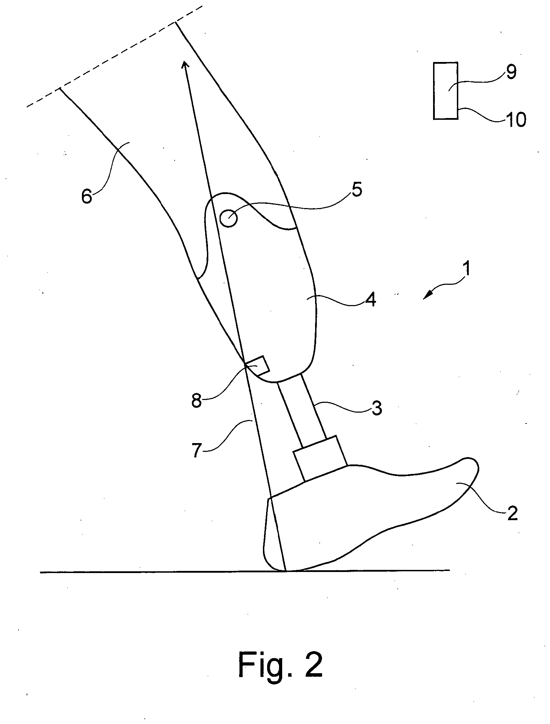

In der Figur 2 ist die Maximalstellung des Prothesenfußes 2 in posterior-Richtung gezeigt, bei einem Fersenauftritt verläuft der Vektor der Bodenreaktionskraft 7 hinter der Kniedrehachse 5, so dass es zu einer sehr starken Beugung aufgrund des flektierenden Kniemomentes kommt. Diese ungewollte Kniebeugung muss der Patient mit dem versorgten Bein abfangen, was nachteilig ist. Daher wird der Orthopädietechniker den Winkelverlauf nach dem Fersenauftritt analysieren und feststellen, dass es zu einer zu starken oder zu schnellen Kniebeugung und damit zu einer zu schnellen Verlagerung des Schaftes 4 relativ zur Vertikalen kommt. Dies führt dazu, dass der Prothesenfuß 2 weiter anterior verschoben werden muss, bis der Patient beginnt, das Knie kontrolliert zu beugen.

Die Bestimmung des Aufbaus über den Fersenauftritt wird erleichtert, wenn der Kniewinkel als Messwert vorliegt, so dass zusätzliche Inertialwinkelsensoren 8 an dem Oberschenkel angeordnet werden müssten. Der Kniewinkel wird durch die Differenzbildung der Segmentwinkel aus Oberschenkel und Unterschenkel in der Sagittalebene berechnet. Anhand der gemessenen Kniewinkelkurve oder gegebenenfalls auch allein anhand der gemessenen Unterschenkelkurve für mehrere Schritte kann nun eine Empfehlung darüber gegeben werden, in welche Richtung der Prothesenfuß verschoben werden muss. Diese Empfehlung kann über eine Ausgabeeinrichtung 10 an einem Komparator 9, der mit dem Sensor 8 oder den Sensoren 8 in Verbindung steht, erfolgen. Innerhalb des Komparators 9 werden die gemessenen Winkel verarbeitet und der Ausgabebefehl oder Ausgabewert gegebenenfalls anhand von Sollkurven oder gemessenen Winkelwerten des kontralateralen, unversorgten Beines ermittelt.

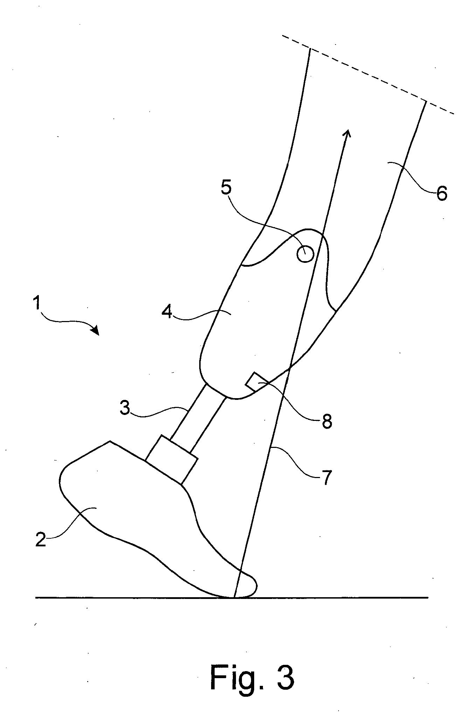

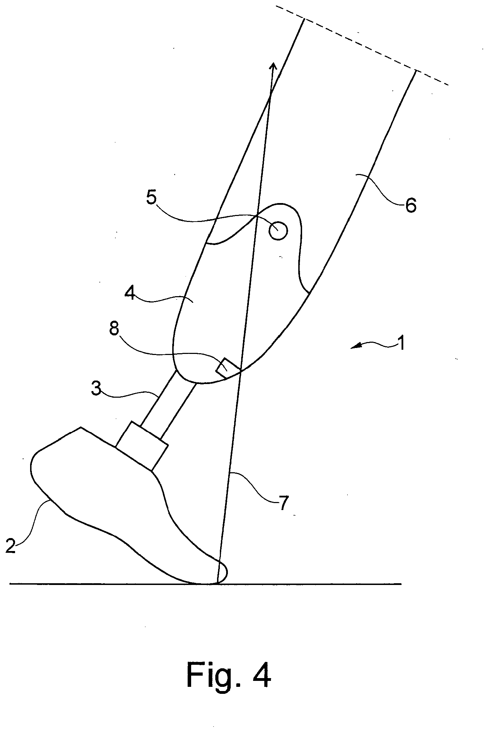

Eine weitere Indikation, ob der Prothesenaufbau zutreffend ist oder nicht, kann in der Phase der Zehenablösung, des sogenannten "toe-off" erfolgen, die in den Figuren 3 und 4 dargestellt ist. Bei einer Orientierung des Prothesenfußes 2 zu weit anterior gemäß Figur 3 wird ein kniestreckendes Moment aufgrund des Verlaufes der resultierenden Bodenreaktionskraft 7 anterior der Kniedrehachse 5 ausgeübt, so dass ein Einbeugen nach dem toe-off nicht oder nur schwer möglich ist. Dementsprechend ist es notwendig, den Prothesenfuß 2 weiter posterior zu positionieren.

Ist der Prothesenfuß 2 zu weit posterior, wie er in der Figur 4 gezeigt ist, verläuft der resultierende Bodenreaktionskraftvektor 7 beim toe-off posterior der Kniedrehachse 5, was zu einem plötzlichen und unkontrollierten Einbeugen führt oder zu einer erhöhten Belastung des Patienten bei einer transtibialen Prothese. Wird beim toe-off oder kurz danach keine Kniebeugung festgestellt bzw. keine ausreichende Vergrößerung des Inertialwinkels des Schaftes 4 und des Oberschenkels, ist von einer Positionierung zu weit anterior auszugehen, bei einer schnellen Winkelvergrößerung und einem sehr weiten Durchschwingen sowie einem plötzlichen Einbeugen kann von einer Positionierung zu weit posterior ausgegangen werden, so dass eine entsprechende Verstellung notwendig wird, um dies auszugleichen. Über die Ausgabeeinrichtung 10 wird auf der Grundlage der ermittelten Werte ein Signal ausgegeben, ob der Aufbau den Vorgaben entspricht oder ob Änderungen und welche Änderungen vorgenommen werden sollen, also ob eine Verschiebung anterior oder posterior erfolgen soll.

Ein physiologisch korrekter Gang ist unter anderem dadurch gekennzeichnet, dass die Segmentwinkel von Oberschenkel und Unterschenkel beider Beine beim normalen Gehen in der Ebene einen nahezu identischen Bewegungsablauf und Bewegungsumfang beschreiben. Wenn bei einem Prothesenträger der prothesenseitige Oberschenkelwinkel in der Sagittalebene einen reduzierten Bewegungsumfang aufweist, kann dies ein Hinweis auf eine während des Prothesenaufbaus nicht berücksichtigte Flexionskontraktur sein. Wird also während des Gehens der Bewegungsumfang des rechten und linken Oberschenkels an dem Patienten über Intertialsensoren direkt gemessen, ist es möglich, unterschiedliche Bewegungsumfänge aufgrund des abweichenden Inertialwinkels während eines Gangzyklus' bzw. eines Schrittes zu ermitteln. Sofern der prothesenseitige Bewegungsumfang geringer ausfällt, wird über den Komparator 9 und die Ausgabeeinrichtung 10 ein Hinweis dahingehend ausgegeben, dass eine Flexionskontraktur vorliegt, so dass der Prothesenaufbau entsprechend verändert werden muss.

Eine Anordnung eines Inertialwinkelsensors 10 dergestalt, dass eine Beckenkippung in der Frontalebene beim Gehen ermittelt werden kann, kann bei Überschreiten der vorgegebenen Winkel der Prothesenaufbau durch eine weitere Adduktion des Schaftes 4 optimiert werden.

Wenn während der Schwungphase das Unterschenkelrohr 3 bzw. der Schaft 4 der versorgten Seite eine Rotationsschwingung erfährt, ist dies ein Hinweis auf einen falsch eingestellten Außenrotationswinkel der Kniedrehachse 5. Dies bedeutet, dass die Kniedrehachse 5 bei transfemuralen Prothesen nicht orthogonal zu der Bewegungsrichtung des Oberschenkels verläuft, so dass der Unterschenkel eine dynamische Unwucht erfährt. Wird eine Unwucht aufgrund der hohen Schwingfrequenz des gebeugten Unterschenkels gemessen, ist dies ein Hinweis auf eine fehlerhafte Ausrichtung der Kniedrehachse 5, so dass eine entsprechende Korrektur des Aufbaus vorgenommen werden kann.

Bei Transtibialprothesen kann eine ausreichende Beugung des Kniegelenks vorliegen, wenn die Kniebeugung größer als 5° vorhanden ist, wenn die Kniewinkelgeschwindigkeit kleiner als 100°/s beträgt und die Streckung des Knies in der Standphase größer als die Hälfte des Kniebeugewinkels ist. Wird keine ausreichende Beugung im Kniegelenk festgestellt, ist der Prothesenfuß 2 schrittweise nach anterior zu positionieren, bis eine gut kontrollierbare Bewegung des Knies stattfindet. Die Feststellung bezüglich des annähernd physiologischen Kniewinkels kann über die Ausgabeeinrichtung 10 durch ein akustisches Signal erfolgen, bei Messwerten außerhalb festgestellter Parameter oder der Symmetriewerte des kontralateralen Beins kann ein negativ klingendes Signal ausgegeben oder eine optische Warneinrichtung aktiviert werden.

Bei Transfemuralprothesen kann es vorkommen, dass sich eine Hüftflexionskontraktur beim Gehen stärker bemerkbar macht als in der Anamnese. Um dies zu ermitteln, wird die Messung der Oberschenkelwinkelbewegung beim Gehen prothesenseitig und kontralateral in der Sagittalebene durchgeführt. Bei einer geringeren Bewegung auf der Prothesenseite, beispielsweise kleiner als 70% der Winkelwerte für die kontralaterale Seite, wird die Schaftflexion innerhalb der Prothese erhöht, bis das Bewegungsausmaß angeglichen ist.

Eine weitere Problematik bei Transfemuralprothesen kann darin liegen, dass die Leistungsfähigkeit des gluteus medius schwächer als in der Anamnese festgestellt ist. Die Außenrotation der Knieachse ist dann geringer als für den individuellen Gehstil eigentlich erforderlich ist. Um hier Fehler beim Prothesenaufbau festzustellen, wird der Beckenwinkel beim Gehen gemessen. Wird ein Kippen der Hüfte zur Positionierung des Körperschwerpunktes über die Prothese oder ein Absinken der Hüfte auf der kontralateralen Seite gemessen, beispielsweise größer als 5° zur Horizontalen, wird der Abduktionswinkel schrittweise erhöht, bis der Patient beim Gehen eine Beckenkippung im Bereich von ± 5° nicht mehr verlässt.

Zur Rotation des Schaftes 4 um dessen Längsachse bzw. der Pendelbewegung des Unterschenkels in der Frontalebene wird ebenfalls eine Inertialwinkelmessung am Unterschenkel durchgeführt. Wird beim Gehen in der Ebene eine Winkelbewegung beispielsweise größer als 5° gemessen, wird die Knieachse 5 schrittweise nach außen rotiert, bis die Winkelbewegung in einem tolerierbaren Bereich verbleibt.

## Patentansprüche

1. Verfahren zur Bestimmung von Fehlstellungen eines Prothesenfußes (2) relativ zu einem Unterschenkelrohr (3) im Aufbau von Prothesen der unteren Extremitäten mit folgenden Schritten:
- Ermittelung von Inertialmessdaten und/oder der daraus abgeleiteten Größen einer versorgten Extremität über zumindest einen Inertialsensor (8), der an einer Komponente (2, 3, 4) der Prothese der unteren Extremität angeordnet und mit einem Komparator (9) gekoppelt ist, über zumindest einen Gangzyklus und
- Vergleichen der ermittelten Inertialmessdaten und/oder der daraus abgeleiteten Größen mit Sollwerten in dem Komparator (9)
**dadurch gekennzeichnet, dass** aus einem gemessenen Absolutwinkel des Unterschenkels und einem gemessenen Absolutwinkel eines Oberschenkels ein Kniewinkel bestimmt und mit Sollwerten verglichen wird.

2. Verfahren nach Anspruch 1, **dadurch gekennzeichnet, dass** als Inertialmessdaten Absolutwinkel, Linearbeschleunigungen und/oder Drehraten gemessen werden.

3. Verfahren nach Anspruch 1 oder 2, **dadurch gekennzeichnet, dass** sowohl an der mit der Prothese versorgten als auch an der unversorgten korrespondierenden Extremität die jeweiligen Inertialmessdaten und/oder daraus abgeleitete Größen ermittelt werden.

4. Verfahren nach einem der voranstehenden Ansprüche, **dadurch gekennzeichnet, dass** eine Abweichungsmeldung bei Abweichungen der ermittelten Winkel von den Sollwerten und/oder Winkeln der korrespondierende unversorgten Extremität oder eine Bestätigungsmeldung bei Werten innerhalb der Sollwerte und/oder der ermittelten Werte für die korrespondierende unversorgte Extremität ausgegeben wird.

5. Verfahren nach einem der voranstehenden Ansprüche, **dadurch gekennzeichnet, dass** ein Beckenwinkel in der Frontalebene über zumindest einen Inertialwinkelsensor ermittelt und bei Überschreiten eines Grenzwertes eine Fehlermeldung ausgegeben wird.

6. Verfahren nach einem der voranstehenden Ansprüche, **dadurch gekennzeichnet, dass** die Inertialmessdaten über mehrere Gangzyklen ermittelt werden.

## Claims

1. A method for determining incorrect positions of a prosthetic foot (2) relative to a lower leg tube (3) in the set-up of prostheses of the lower extremities, comprising the following steps:
- establishing inertial measurement data and/or the variables derived therefrom, of a treated extremity by means of at least one inertial sensor (8) which is arranged on a component (2, 3, 4) of a prosthesis of the lower extremity and coupled to a comparator (9), over at least one gait cycle and
- comparing the established inertial measurement data and/or the variables derived therefrom with intended values in the comparator (9),
**characterized in that** a knee angle is determined from the measured absolute angle of the lower leg and the measured absolute angle of the thigh and compared to intended values.

2. The method as claimed in claim 1, **characterized in that** absolute angles, linear accelerations and/or angular rates are measured as inertial measurement data.

3. The method as claimed in claim 1 or 2, **characterized in that** the respective inertial measurement data and/or variables derived therefrom are established both on the extremity treated with the prosthesis and on the untreated corresponding extremity.

4. The method as claimed in one of the preceding claims, **characterized in that** a deviation message is output in the case of deviations between the established angle and intended values and/or angles of the corresponding untreated extremity or a confirmation message is output in the case of values within the intended values and/or the established values for the corresponding untreated extremity.

5. The method as claimed in one of the preceding claims, **characterized in that** a pelvic angle in the frontal plane is established by at least one inertial angle sensor and an error message is output if a limit value is exceeded.

6. The method as claimed in one of the preceding claims, **characterized in that** the inertial measurement data are established over a plurality of gait cycles.

## Revendications

1. Procédé de détermination de défauts d'alignement d'un pied prothétique (2) par rapport à un tube de jambe inférieure (3) dans la structure de prothèses des membres inférieurs, comprenant les étapes suivantes consistant à :
- déterminer, par l'intermédiaire d'au moins un capteur inertiel (8) disposé sur un composant (2, 3, 4) de la prothèse du membre inférieur et couplé à un comparateur (9), des données de mesure inertielle et/ou des grandeurs, dérivées de celles-ci, d'un membre équipé, sur au moins un cycle de marche, et
- comparer les données de mesure inertielle déterminées et/ou les grandeurs dérivées de celles-ci à des valeurs de consigne dans le comparateur (9),
**caractérisé en ce qu'**un angle de genou est déterminé à partir d'un angle absolu mesuré de la jambe inférieure et d'un angle absolu mesuré d'une cuisse, et est comparé à des valeurs de consigne.

2. Procédé selon la revendication 1,
**caractérisé en ce que** des angles absolus, des accélérations linéaires et/ou des vitesses de rotation sont mesurés comme données de mesure inertielle.

3. Procédé selon la revendication 1 ou 2,
**caractérisé en ce que** les données de mesure inertielle respectives et/ou les grandeurs dérivées de celles-ci sont déterminées aussi bien sur le membre équipé de la prothèse que sur le membre correspondant non équipé.

4. Procédé selon l'une des revendications précédentes,
**caractérisé en ce qu'**un message d'écart est émis en cas d'écarts des angles déterminés par rapport aux valeurs de consigne et/ou aux angles du membre correspondant non équipé, ou un message de confirmation est émis en cas de valeurs comprises dans les valeurs de consigne et/ou dans les valeurs déterminées pour le membre correspondant non équipé.

5. Procédé selon l'une des revendications précédentes,
**caractérisé en ce qu'**un angle du bassin dans le plan frontal est déterminé par au moins un capteur d'angle inertiel, et un message d'erreur est émis en cas de dépassement d'une valeur limite.

6. Procédé selon l'une des revendications précédentes,
**caractérisé en ce que** les données de mesure inertielle sont déterminées sur plusieurs cycles de marche.
